# EUROPEAN PATENT APPLICATION

(11) **EP 4 385 444 A1**
(43) Date of publication of application: **19.06.2024**
(21) Application number: 22213901.6
(22) Date of filing: 15.12.2022
(51) Int. Cl.: A61B 18/18

(54) **IPL SKIN CARE OR HAIR CARE DEVICE**

(71) Applicant: Koninklijke Philips N.V., 5656 AG Eindhoven (NL)
(72) Inventor: WRIGHT, Christopher John, 5656AG Eindhoven (NL); WALKER, Nicholas Simon, 5656AG Eindhoven (NL); HALPERN PASTOR, Alejandro, 5656AG Eindhoven (NL)
(74) Representative: Philips Intellectual Property & Standards

(57) **Abstract**

An intense pulse light, IPL, skin or hair care device within which a cooling arrangement is provided. The cooling arrangement comprises a phase change material configured to transition from a first phase to a second phase responsive to heat emitted by the light source.

## Description

### FIELD OF THE INVENTION

This invention relates to Intense Pulse Light, IPL, hair or skin treatment devices.

### BACKGROUND OF THE INVENTION

Light therapy is known for hair removal and hair growth reduction.

Home-use photo-epilation consumer devices are commercially available, such as the Lumea (TM) of Philips (TM). Home-use devices typically take the form of a hand-held device using Intense Pulsed Light (IPL) technology from e.g. a Xenon flash lamp at a relatively low fluence (e.g., 6.5 J/cm²), as compared to professional devices for permanent photo-epilation, that use fluences in excess of 10 J/cm².

Light is absorbed by the hair roots and the hair follicles present in the skin, which are considerably heated as a result of the relatively high energy density of the light. By using a proper configuration of the light energy, namely the wavelength, intensity, and pulse duration, selective heating of the hair root and the desired temporary or permanent damage to the hair follicle can be achieved.

The IPL technology uses the flash lamp to deliver an intense, visible, broad-spectrum pulse of light, generally in the visible spectral range of 400 to 1200 nm. Cutoff filters are for example used to selectively filter out shorter wavelengths, especially potentially damaging ultraviolet light. The resulting light has a spectral range that targets specific structures and chromophores, in particular the melanin pigment in hair. IPL shares some similarities with laser treatments, in that they both use light to heat and destroy their targets. Unlike lasers that use a single wavelength of light which typically matches only one chromophore and hence only treats one condition, IPL uses a broad spectrum.

It is common for an IPL light sources to overheat, and this can reduce the effective and/or lifespan of the IPL device. Thus, there is a need for an IPL device to include an effective and appropriate cooling arrangement.

### SUMMARY OF THE INVENTION

The invention is defined by the claims.

According to examples in accordance with an aspect of the invention, there is provided an IPL skin or hair care device, comprising:
an optical system for delivering IPL light to the skin of a user for a skin or hair care function, the optical system comprising a light source configured to generate IPL light; and
a cooling arrangement for cooling the optical system,
wherein the cooling arrangement comprises a phase change material, PCM, configured to transition from a first phase to a second phase responsive to heat emitted by the light source.

The invention proposes the use of a PCM to cool an IPL device. In particular, it is proposed that a PCM may be employed in an IPL device and configured to transition from a first phase to a second phase in order to absorb heat emitted by the light source (as a result of generating IPL light). That is, it is proposed that an IPL device's components may be cooled using a PCM.

By employing a PCM to absorb heat by transitioning from a first phase to a second phase, embodiments may reduce or prevent overheating of an IPL device, which mat in turn improve the effectiveness and/or lifetime of the IPL device.

Also, unlike conventional cooling mechanisms (cannot be included on the front side of the light source), embodiments may permit the provision of the cooling arrangement on the front side of the light source (i.e. in the optical path) without impeding the generated IPL light from reaching a user's skin during use.

Embodiments may thus be based on the realization that a PCM may be employed to absorb heat via phase transition , wherein the phase transition is caused by heat generated by the light source. That is, the PCM may be chosen/configured so that it undergoes a phase transition responsive to the light source generating IPL light, and the phase transition may then absorb heat generated by the light source.

The optical system may comprise an IPL light exit window through which generated IPL light is output for application to the skin of the user. The PCM may then be positioned on an optical path along which the generated IPL light travels from the light source to the IPL exit window so that generated IPL light is incident on the PCM. Further, the PCM may be transparent. In this way, embodiments may facilitate the provision of a cooling arrangement on the 'front' side of the IPL device (i.e. the side of the device which is configured to output IPL light for application to a user).

The cooling arrangement may comprise a carrier material configured to support the PCM. By way of example, the carrier material may comprise a porous material that is configured to permit vapourisation and escape of the PCM from the carrier material.

The carrier material may comprise a thermally conductive transparent structure or matrix. The PCM may then be supported or suspended by/in the structure/matrix transparent gel. For instance, the carrier material may comprise a rigid porous structure or a semi-rigid organic polymer-based substance, such as an aerogel. The PCM may thus be contained within a thermally conductive aerogel structure. A physical carrier structure may therefore be provided hold the PCM within a matrix for example. In this way, heat may be efficiently transferred through the depth of the PCM.

A proposed arrangement for front side cooling of a skin treatment IPL device may therefore comprise a thermally conductive transparent gel that includes PCM). This may be positioned between the light source and a treatment window (e.g. IPL exit window) of the IPL device, thus providing an optically transmissive front-side PCM-based cooling system.

By way of example, the transparent gel may comprise an aerogel. Preferably, the aerogel may comprise a hexagonal Boron Nitride, hBN, aerogel. BN aerogel, often called 'White graphene', has very high surface area, thermal conductance, and very low light absorbance properties. For instance, the material nanostructure scatters light very strongly and hardly absorbs it (<1% absorbance). Also, hBN aerogels may be used as a sponge or wick for oils and solvents.

The optical system may comprise a heat sink configured to distribute heat from the light source. The carrier material may then be configured to be in contact with at least one surface of the heat sink. In this way, heat from the light source may be efficiently transferred to the carrier material and PCM, thereby improving the cooling process.

The first phase may be a liquid phase and the second phase a vapour phase. For such embodiments, the cooling arrangement may comprise one or more wicking structures upon which the PCM is configured to condense to transition from the second phase to the first phase. That is, the PCM may be a liquid to vapour working fluid, and wicking structures may be provided to condense the PCM and wick it back to the transparent evaporator.

In some embodiments, the PCM may be non-mobile, such as a solid-liquid phase transition material, or liquid-vapour where the aerogel and associated structures prevent vapour release (e.g. the aerogel may be bounded and sealed).

Some embodiments may comprise a housing within which the cooling arrangement is housed. The housing may be sealed to prevent vapour loss from the housing.

For instance, a sealed chamber may be provided to contain the PCM so that its loss from the IPL device is prevented.

Some exemplary embodiments may comprise a reflective structure configured, in use, to reflect scattered or reflected IPL light towards the skin of a user. The PCM and/or carrier material may be highly scattering and non-absorbent of IPL light. The provision of one or more reflective structures may thus help to ensure that IPL light scattered by the PCM and/or carrier material is reflected back to the skin of the user, thereby improving efficiency of the IPL device.

The PCM may be configured to transition from the first phase to the second phase within a temperature range of 20-60 degrees. A range of working PCM fluids can be used which have a critical point in the desired range without application of pressure.

The light source preferably comprises a semiconductor light source array.

One or more concepts are provided for cooling an IPL skin or hair care device. By way of example, proposed embodiments may address the issue of excessive heat bine generated by IPL light emission from a light source. It will therefore be understood that the proposed concept(s) may be employed in a wide range of IPL skin or hair care devices.

The IPL device may, for example, be a photo-epilator device.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an intense pulse light, IPL, skin or hair care device according to an embodiment;
Fig. 2 shows a cross-sectional view of an IPL device according to another embodiment; and
Fig. 3 illustrates an IPL device cooling process according to a proposed embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an intense pulse light, IPL, skin or hair care device in which a phase change material, PCM, it provided for absorbing heat that is emitted as a result of the IPL device generating IPL light. The PCM is configured to transition from a first phase to a second phase responsive to heat emitted by the light source(s) of the IPL device. Thus, it is proposed that an IPL device may be cooled using a PCM.

In particular, front-side cooling may be enabled by using an optically transmissive but highly thermally conductive aerogel layer containing a transparent PCM.

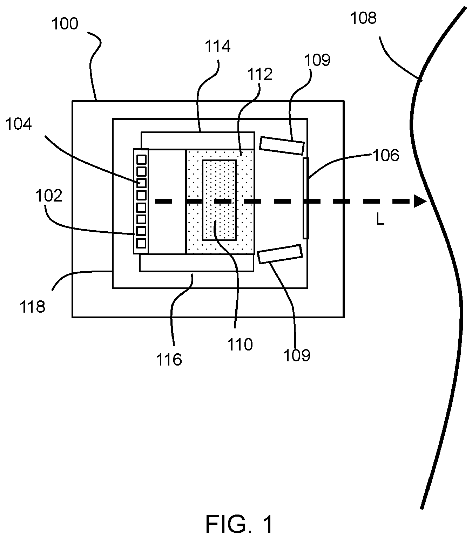

Fig. 1 shows an intense pulse light, IPL, skin or hair care device 100 according to an exemplary embodiment. The IPL device 100 comprises an optical system 102 for delivering IPL light to the skin of a user for a skin or hair care function. The optical system 102 comprises a light source 104 configured to generate IPL light. Specifically, the light source 104 comprises a 2D array of semiconductor light source elements 104, such as LEDs, semiconductor lasers, or VCSELs. The light source elements 104 may be individually addressable.

The optical system 102 also comprises an IPL light exit window 106 through which generated IPL light is output for application to the skin 108 of the user, as illustrated by the dashed arrow labeled "L". Reflective structures 109 are also provided to reflect scattered or reflected IPL light towards light exit window 106 (and thus towards the skin 108 of the user).

The IPL device 100 also comprises a cooling arrangement for cooling the optical system. Specifically, the cooling arrangement comprises a phase change material, PCM 110 (such as ethanol or methanol) configured to transition from a first phase to a second phase responsive to heat emitted by the light source. The PCM 110 is supported by a carrier material 112.

In this embodiment, the carrier material 112 comprises a thermally conductive transparent gel, and the PCM 110 is suspended in the transparent gel. More specifically, the transparent gel comprises an aerogel, namely a hexagonal Boron Nitride, hBN, aerogel.

The PCM 110 is transparent and is positioned on an optical path L along which the generated IPL light travels from the light source 104 to the ILP exit window 1060. In this way, generated IPL light is incident on the PCM 110 and is transmitted through the PCM 110.

The optical system 102 also comprises first 114 and second 116 heat sinks that are configured to distribute heat from the light source 104. The heat sinks 114 and 116 each contact the light source 104 and extend away from the light source 104 so as to also contact the carrier material 112 on opposite side. The carrier material 112 therefore extends across the optical path L between the first 114 and second 116 heat sinks, so that the carrier material spans/bridges a gap between the first 114 and second 116 heat sinks. Heat from the light source 104 is distributed away from the light source 104 by the heat sinks and (thermally) conducted to the carrier material 112 (and the PCM 110 suspended therein).

The PCM 110 is configured to transition from the first phase to the second phase within a temperature range of 20-60 degrees Celsius. Specifically, the PCM transitions between a liquid phase and a vapour phase, for temperatures in the range of 30-50 degrees Celsius.

In the example of Fig. 1, the cooling arrangement also comprises wicking structures (not shown) upon which the PCM 110 is configured to condense so as to transition from the vapour phase to the liquid phase.

To prevent vapour loss, the light source 102 and cooling arrangement is contained within a sealed (e.g. watertight/airtight) housing.

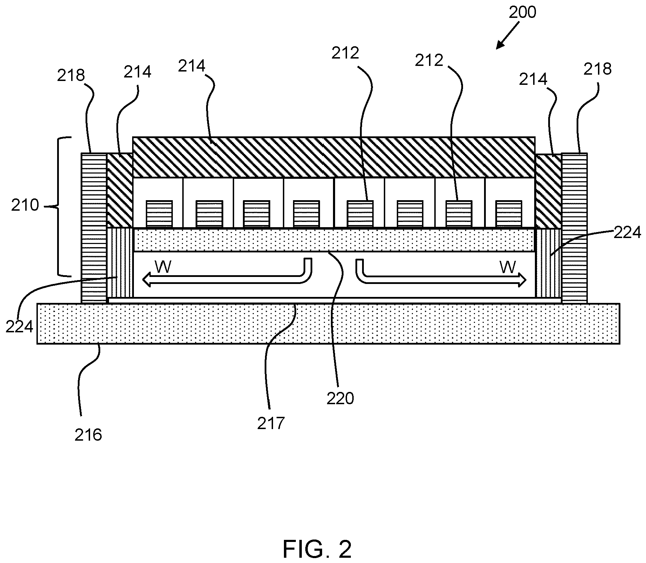

Referring now to Fig. 2, there is depicted a cross-sectional view of an IPL skin or hair care device 200 according to another embodiment.

The IPL device 200 comprises an optical system 210 for producing IPL light which, as a byproduct, creates heat. The optical system 210 comprises light sources 212 configured to generate IPL light. The light sources 212 comprise a layer of micro-LEDs, transparent LEDs, OLEDs, or other suitable technologies.

In this embodiment, the optical system 210 also comprise reflective structures 214 that are configured to reflect light (e.g. emitted light, scattered light or light reflected by the user's skin 216) so that is directed towards the user's skin 216 (through a transparent window 217 of the device 200). This helps to increase the efficiency of the device 200.

The optical system 210 also includes heat sinks 218 for distributing heat which is emitted by the light sources 212.

To enable material phase change-based cooling for the optical system, without significantly affecting the optical efficiency, the device 200 comprises a PCM suspended in a carrier material 220 which has a state transition from a low to high entropy state that occurs as a result of the heat emitted from the light sources 212.

In this embodiment, the PCM 220 comprises a liquid to vapour phase change material, such as ethanol or methanol. However, in other embodiments, solid to liquid or solid to solid PCMs may be implemented.

The carrier material 220 is configured to support/hold the PCM within a matrix such that the PCM is wicked over the surface of the light sources 212, and so that heat it heat is efficiently transferred through the depth of the PCM. This is advantageous because some PCM materials may have a low thermal conductivity, and therefore without the carrier material, only a thin layer of PCM may be viable.

The carrier material 220 comprises a material which is more thermally conductive than the PCM. The carrier material 220 is also porous, so that it can perform a wicking action and allow vapourisation/escape of the PCM from the matrix of the carrier material 220. The carrier material is also transparent (or at least exhibits low optical absorbance), so that light from the light sources is transmitted through the carrier material towards the user's skin 216 (through the transparent window 217 of the device 200).

In this exemplary embodiment, the carrier material comprises a BN aerogel material. This has the following advantages: highly thermally conductive (~400 W m⁻¹ K⁻¹); highly scattering (which produces an even distribution of light); low optical absorbance (e.g. <1% absorbance); high surface area to volume ratio (enabling fast conductance of the heat into the PCM (900 m² g⁻¹); high porosity (allowing a high amount of PCM to be held in a small volume, circa 99.99 % porosity).

The device 200 also comprise wicking structures 224 that are configured to to enable the PCM to condense once vapourised at a location separated from the light sources 212, thereby transferring heat away from the light source 211 (as depicted by the arrows laeblled "W"). The wicking structures 224 may be formed from standard materials which are commonly used in vapour chambers, such as copper foam. The wicking structures 224 are in thermal contact with heat sinks 218 so as to transfer the heat to the outer body of the device 200 (e.g. for dissipation to the ambient environment).

It is noted that the transparent window 217 of the device 200 allows passage of the light out of the device 200, onto the user's skin 216, and also enclose the cooling structures, such that PCM vapour remains within a sealed chamber. The transparent window 217 may be a simple, clear glass window.

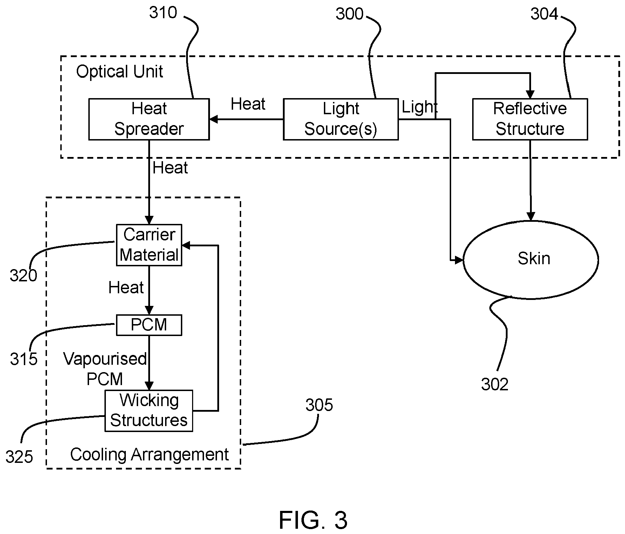

Referring now to Fig. 3, an exemplary IPL device cooling process according to the proposed concept(s) may be summarized as follows;
(A) The light source(s) 300 is/are actuated while the device (e.g. the transparent exit window) is in contact with the user's skin 320, directly through the transparent exit window or reflected back through the transparent exit window by the reflective structure 304.
(B) Light is transmitted to the skin
(C) Heat is transmitted to the cooling arrangement 305 via a heat spreader 310.
(D) The heat is transmitted to the volume of PCM 315 via a carrier material 320.
(E) The heat causes the PCM 315 to change phase.
(F) The PCM 315 evaporates to create a vapour in the chamber, which subsequently condenses onto the wicking structure(s) 325.
(G) Heat is carried away by conventional heat sinking structures so as to be dissipated to the outside of the device (or may be deposited in a thermal mass within the device).
(H) The liquid phase PCM is returned to the carrier material 320 via the wicking structures 325.

Although embodiments have been described above as employing a liquid to vapour PCMs, other embodiments may employ alternative PCMs which employ solid-to-solid or solid-to-liquid transitions.

In some embodiments, a PCM may be used which is static (as in it does not move freely or away from the surface of a heat spreader). For example, these may include:
- A solid-solid PCM, such as A Ni-Ti alloy. However, this may be less preferable due to the lower entropy changes involved between the material states.
- A solid-liquid PCM such as paraffin. The liquid may be: unconstrained (e.g. a pure layer of PCM) or held within a matrix (such as a PCM carrier material). This may be a closed or open pore matrix, as no vapour escape is required.
- A liquid-vapour PCM, wherein the vapour is constrained within the carrier material in a closed-pore embodiment, or as encapsulated inclusions in a stronger transparent solid matrix. This may be less preferable due to the sacrifice of PCM volume which may be included.

A IPL device cooling system is proposed which employs a PCM to absorb heat from the system (which occurs as a result of IPL production). In some embodiments, a front-side cooling system is proposed which enables increased cooling in addition to that which is achievable via only back-side cooling. A transparent and highly scattering material is proposed for front-side cooling which provides improved performance in terms of both optical and thermal criteria.

By way of example, the following advantages may be provided by proposed embodiments:
(i) Utilizing a carrier for the PCM enables a larger volume of PCM to be used due to the high thermal conductivity of the carrier;
(ii) Better cooling enables longer LED lifetime and therefore device effectiveness lifetime; and improved emission efficiency during pulse, increasing device effectiveness.
(iii) Increased output light uniformity.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An intense pulse light, IPL, skin or hair care device (100), comprising:
an optical system (102) for delivering IPL light to the skin of a user for a skin or hair care function, the optical system comprising a light source (104) configured to generate IPL light; and
a cooling arrangement for cooling the optical system,
wherein the cooling arrangement comprises a phase change material (110), PCM, configured to transition from a first phase to a second phase responsive to heat emitted by the light source.

2. The IPL device of claim 1, wherein the optical system (102) comprises an IPL light exit window (106) through which generated IPL light is output for application to the skin of the user,
and wherein the PCM (110) is positioned on an optical path (L) along which the generated IPL light travels from the light source to the IPL exit window so that generated IPL light is incident on the PCM.

3. The IPL device of claim 2, wherein the PCM (110) is transparent.

4. The IPL device of any of claims 1 to 3, wherein the cooling arrangement comprises a carrier material (112) configured to support the PCM (110).

5. The IPL device of claim 4, wherein the carrier material (112) comprises a porous material that is configured to permit vapourisation and escape of the PCM (110) from carrier material.

6. The IPL device of claim 4 or 5, wherein the carrier material (112) comprises a thermally conductive transparent gel and wherein the PCM is suspended in the transparent gel.

7. The IPL deice of claim 6, wherein the transparent gel comprises an aerogel, and preferably wherein the aerogel comprises a hexagonal Boron Nitride, hBN, aerogel.

8. The IPL device of any of claims 4 to 7, wherein the optical system (102) comprises a heat sink (114) configured to distribute heat from the light source (104), and wherein the carrier material contacts at least one surface of the heat sink.

9. The IPL device of any of claims 1 to 8, wherein the first phase is a liquid phase and the second phase is a vapour phase, and wherein the cooling arrangement comprises one or more wicking structures (224) upon which the PCM is configured to condense to transition from the vapour phase to the liquid phase.

10. The IPL device of any of claims 1 to 9, further comprising a housing (118) within which the cooling arrangement is housed, the housing being sealed to prevent vapour loss from the housing.

11. The IPL device of any of claims 1 to 10, further comprising a reflective structure (109) configured, in use, to reflect scattered or reflected IPL light towards the skin (108) of a user.

12. The IPL device of any of claims 1 to 11, wherein the PCM is configured to transition from the first phase to the second phase within a temperature range of 20-60 degrees Celsius.

13. The device of any of claims 1 to 12, wherein the light source (104) comprises a semiconductor light source array.
